# FASCICULE DE BREVET EUROPEEN

(11) **EP 0 981 340 B1**
(45) Date de publication et mention de la délivrance du brevet: **12.12.2001**
(21) Numéro de dépôt: 98909550.0
(22) Date de dépôt: 17.02.1998
(51) Int. Cl.: A61K 31/14, A61K 31/46, A61K 9/16

(54) **FORMULATIONS PHARMACEUTIQUES PRESENTEES SOUS FORME SECHE POUR L'ADMINISTRATION ORALE D'UN COMPOSE AMMONIUM QUATERNAIRE CYCLIQUE**
ARZNEIZUBEREITUNGEN IN TROCKNER FORM ZUR ORALEN VERABREICHUNG EINER CYCLISCHEN QUATERNÄREN AMMONIUMVERBINDUNG
PHARMACEUTICAL FORMULATIONS IN DRY FORM FOR THE ORAL ADMINISTRATION OF A CYCLIC QUATERNARY AMMONIUM COMPOUND

(30) Priorité: 17.02.1997 FR 9701826
(43) Date de publication de la demande: 01.03.2000
(73) Titulaire: SANOFI-SYNTHELABO, 75013 Paris (FR)
(72) Inventeur: ABRAMOVICI, Bernard, F-34990 Juvignac (FR); BOULENC, Xavier, F-34080 Montpellier (FR); GAUTIER, Jean-Claude, F-34830 Clapiers (FR); VILAIN, Pol, F-34570 Saussan (FR)
(74) Mandataire: Gillard, Marie-Louise
(86) Numéro de dépôt international: FR9800299
(87) Numéro de publication internationale: WO9835663

(56) Documents cités:
- EP-A- 0 512 901
- EP-A- 0 515 240
- EP-A- 0 559 538
- EP-A- 0 591 040
- EP-A- 0 708 101
- EP-A- 0 723 959
- WO-A-95/26335
- WO-A-95/26339
- WO-A-96/06094
- WO-A-96/12479
- WO-A-96/23787

## Description

La présente invention se rapporte à de nouvelles formulations pharmaceutiques sous forme sèche pour l'administration orale, contenant comme principe actif, un composé ammonium quaternaire cyclique.

En particulier, l'invention concerne des formulations pharmaceutiques pour l'administration orale contenant, comme principe actif, un composé de formule : 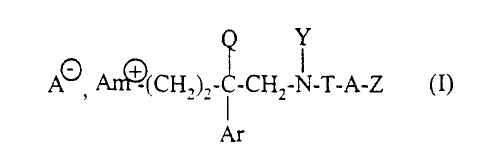 dans laquelle :
- A^{⊖} est un anion pharmaceutiquement acceptable ;
- Am^{⊕} représente :
   i - soit un groupe Am₁^{⊕} de formule : 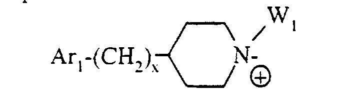 dans laquelle :
      - Ar₁ représente un phényle non substitué ou substitué une ou plusieurs fois par un substituant choisi parmi un atome d'halogène, un hydroxy, un (C₁-C₄)alcoxy, un (C₁-C₄)alkyle ou un trifluorométhyle, lesdits substituants étant identiques ou différents ;
      - x est zéro ou un ;
      - W₁ représente un (C₁-C₆)alkyle ou un groupe benzyle ; le substituant W₁ étant soit en position axiale soit en position équatoriale ;
   ii - soit un groupe Am₂^{⊕} de formule : 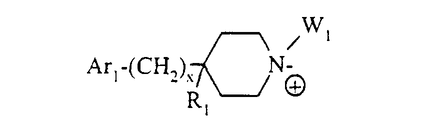 dans laquelle :
      - Ar₁, x et W₁ sont tels que définis ci-dessus ;
      - R₁ représente un hydroxy ; un (C₁-C₄)alcoxy ; un formyloxy ; un (C₁-C₃)alkylcarbonyloxy; un carboxy ; un (C₁-C₄)alcoxycarbonyle ; un cyano ; un (C₁-C₃)alkylcarbonylamino ; un mercapto ; un (C₁-C₄)alkylthio ;
   iii - soit un groupe Am₃^{⊕} de formule : 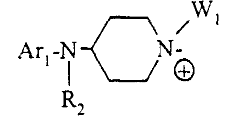 dans laquelle :
      - Ar₁ et W₁ sont tels que définis ci-dessus ;
      - R₂ représente l'hydrogène ; un (C₁-C₃)alkyle ; un (C₁-C₃)alkylcarbonyle ;
   iv - soit un groupe Am₄^{⊕} de formule : 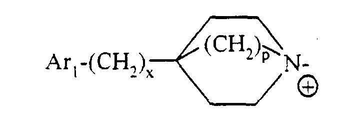 dans laquelle :
      - Ar₁ et x sont tels que définis ci-dessus ;
      - p est un ou deux ;
   v - soit un groupe Am₅^{⊕} de formule : 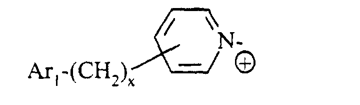 dans laquelle :
      - Ar₁ et x sont tels que définis ci-dessus ;
      - Ar représente un phényle non substitué ou substitué une ou deux fois par un substituant choisi parmi un atome d'halogène, un (C₁-C₃)alcoxy, un (C₁-C₃)alkyle ou un trifluorométhyle, lesdits substituants étant identiques ou différents ; un naphtyle ; un indolyle ;
      - Q et Y représentent l'un des groupes de valeurs suivantes :
         a) Q₁ et Y₁ ;
         b) Q₂ et Y₂ lorsque Am^{⊕} représente un groupe Am₁^{⊕}, Am₂^{⊕}, Am₄^{⊕} ou Am₅^{⊕} ;
         c) Q₃ et Y₃ lorsque Am^{⊕} représente un groupe Am₁^{⊕}, Am₂^{⊕} ou un groupe Am₄^{⊕} dans lequel Ar₁ représente un phényle et p est deux ;
         d) Q₄ et Y₄ lorsque Am^{⊕} représente un groupe Am₁^{⊕}, Am₃^{⊕}, Am₄^{⊕} ou Am₅^{⊕} ;
      - Q₁ représente l'hydrogène ;
      - Y₁ représente l'hydrogène ; un (C₁-C₄)alkyle ; un ω-(C₁-C₄)alcoxy-(C₂-C₄)alkylène ; un ω-(C₁-C₄)alkylcarbonyloxy-(C₂-C₄)alkylène ; un ω-benzoyloxy-(C₂-C₄)alkylène ; un ω-hydroxy-(C₂-C₄)alkylène; un ω-(C₁-C₄)alkylthio-(C₂-C₄)alkylène ; un ω-(C₁-C₄)alkylcarbonyl-(C₂-C₄)alkylène; un ω-carboxy-(C₂-C₄)alkylène ; un ω-(C₁-C₄)alcoxycarbonyl-(C₂-C₄)alkylène ; un ω-benzyloxy-(C₂-C₄)alkylène ; un ω-formyloxy-(C₂-C₄)alkylène ; un ω-R₃NHCOO-(C₂-C₄)alkylène ; un ω-R₄R₅NCO-(C₂-C₄)alkylène ; un ω-R₆CONR₇-(C₂-C₄)alkylène ; un ω-R₈OCONR₇-(C₂-C₄)alkylène ; un ω-R₄R₅NCONR₇-(C₂-C₄)alkylène ; un ω-R₉SO₂NR₇-(C₂-C₄)alkylène ; un ω-cyano-(C₁-C₃)alkylène ;
      - Q₂ et Y₂, ensemble, constituent un groupe éthylène, triméthylène ou tétraméthylène ;
      - Q₃ et Y₃, ensemble, constituent un groupe : 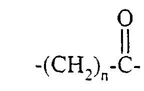 dans lequel n est un, deux ou trois ;
      - Q₄ et Y₄, ensemble, constituent un radical choisi parmi :
         A₁) -O-CH₂-
         A₂) -O-CO-
         A₃) -CH₂-O-CO-
         A₄) -O-CH₂-CO-
         A₅) -O-CH₂-CH₂-
         A₆)-N(R₁₀)-CO-
         A₇) -N(R₁₀)-CO-CO-
         A₈) -N(R₁₀)-CH₂-CH₂-
      - T représente - soit un groupe -CO- lorsque Q et Y représentent Q₁ et Y₁, Q₂ et Y₂ ou Q₄ et Y₄ lorqu'ils constituent, ensemble, un radical A₁), A₅) ou A₈) ;
         - soit un groupe -CH₂- lorsque Q et Y représentent Q₃ et Y₃ ou Q₄ et Y₄ lorsqu'ils constituent, ensemble, un radical A₂), A₃), A₄), A₆) ou A₇) ;
      - A représente soit une liaison directe ou un groupe méthylène lorsque T est -CO-, soit une liaison directe lorsque T est -CH₂- ;
      - Z représente :
         - un phényle non substitué ou substitué une ou plusieurs fois par un substituant choisi parmi : un atome d'halogène ; un trifluorométhyle ; un cyano ; un hydroxy ; un nitro ; un amino non substitué ou substitué une ou plusieurs fois par un (C₁-C₄)alkyle ; un benzylamino ; un carboxy ; un (C₁-C₁₀)alkyle ; un (C₃-C₇)cycloalkyle non substitué ou substitué une ou plusieurs fois par un méthyle ; un (C₁-C₁₀)alcoxy ; un (C₃-C₇)cycloalkyloxy non substitué ou substitué une ou plusieurs fois par un méthyle ; un mercapto ; un (C₁-C₁₀)alkylthio ; un (C₁-C₆)alkylcarbonyloxy ; un (C₁-C₆)alkylcarbonylamino ; un benzoylamino ; un (C₁-C₄)alcoxycarbonyle; un (C₃-C₇)cycloalkylcarbonyle ; un carbamoyle non substitué ou substitué une ou deux fois par un (C₁-C₄)alkyle ; un uréido non substitué ou substitué une ou deux fois en position 3 par un (C₁-C₄)alkyle ou un (C₃-C₇)cycloalkyle ; un (pyrrolidin-1-yl)carbonylamino, lesdits substituants étant identiques ou différents ;
         - un naphtyle non substitué ou substitué une ou plusieurs fois par un halogène, un trifluorométhyle, un (C₁-C₄)alkyle, un hydroxy ou un (C₁-C₄)alcoxy ;
         - un pyridyle ; un thiényle ; un indolyle ; un quinolyle ; un benzothiényle ; un imidazolyle ;
      - R₃ représente un (C₁-C₇)alkyle ou un phényle ;
      - R₄ et R₅ représentent chacun indépendamment un hydrogène ou un (C₁-C₇)alkyle ; R₅ peut de plus représenter un (C₃-C₇)cycloalkyle, un (C₃-C₇)cycloalkylméthyle, un phényle ou un benzyle ; ou bien R₄ et R₅ ensemble avec l'atome d'azote auquel ils sont liés constituent un hétérocycle choisi parmi l'azétidine, la pyrrolidine, la pipéridine, la morpholine, la thiomorpholine, la perhydroazépine et la pipérazine non substituée ou substituée en position 4 par un (C₁-C₄)alkyle ;
      - R₆ représente un hydrogène, un (C₁-C₇)alkyle, un vinyle, un phényle, un benzyle, un pyridyle ou un (C₃-C₇)cycloalkyle non substitué ou substitué par un ou plusieurs méthyles ;
      - R₇ représente un hydrogène ou un (C₁-C₇)alkyle ;
      - R₈ représente un (C₁-C₇)alkyle ou un phényle ;
      - R₉ représente un (C₁-C₇)alkyle ; un amino non substituté ou substitué par un ou deux (C₁-C₇)alkyles ; un phényle non substitué ou substitué une ou plusieurs fois par un substituant choisi parmi : un atome d'halogène, un (C₁-C₇)alkyle, un trifluorométhyle, un hydroxy, un (C₁-C₇)alcoxy, un carboxy, un (C₁-C₇)alcoxycarbonyle, un (C₁-C₇)alkylcarbonyloxy, un cyano, un nitro, un amino non substitué ou substitué par un ou deux (C₁-C₇)alkyles, lesdits substituants étant identiques ou différents ;
      - R₁₀ représente l'hydrogène ou un (C₁-C₄)alkyle ;
ainsi que ses sels éventuels avec des acides minéraux ou organiques et leurs solvates éventuels,
formulée par granulation humide, contenant en pourcentage du poids total de la formulation :
- principe actif 0,5 à 50 %
- liant 1 à 10 %
- agent de désagrégation 0 à 10 %
- antiadhérant 0 à 5 %
- lubrifiant 0,2 à 5 %
- agent d'écoulement 0 à 15 %
- polysorbate 80 4 à 20 %
- colorant 0 à 2 %
- aromatisant 0 à 2 %
- diluant en quantité suffisante (Q.S.) pour 100 %, et contenant de 10 mg à 100 mg de polysorbate 80 par unité de dosage

Les composés de formule (I) utiles pour l'invention comprennent aussi bien les racémiques, les isomères optiquement purs, ainsi que les isomères axiaux et équatoriaux lorsque dans le composé de formule (I), Am^{⊕} représente un groupe Am₁^{⊕}, un groupe Am₂^{⊕} ou un groupe Am₃^{⊕}.

Les composés de formule (I) sont décrits dans les demandes de brevet EP-A-0 512 901, EP-A-0 515 240, EP-A-0 559 538, EP-A-0 591 040, WO 95/26 339, EP-A-0 700 382, EP-A-0 723 959 et WO 96/23 787.

Parmi les composés de formule (I), ceux de formule : 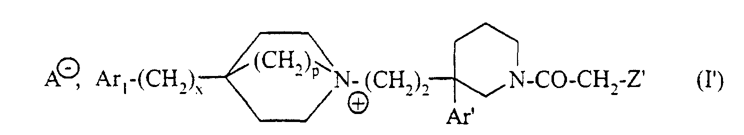 dans laquelle :
- Ar₁, x et p sont tels que définis pour un composé de formule (I) ;
- Ar' représente un 3,4-dichlorophényle ou un 3,4-difluorophényle ;
- Z' représente un phényle substitué en position 3 par un halogène ou un (C₁-C₁₀)alcoxy ;
- A^{⊖} représente un anion pharmaceutiquement acceptable ;
sont préférés pour l'invention.

Plus particulièrement, l'invention se rapporte à des formulations pharmaceutiques sous formes sèches pour administration orale du (S)-1-{2-[3-(3,4-dichlorophényl)-1-(3-isopropoxyphénylacétyl)pipéridin-3-yl]éthyl}-4-phényl-1-azoniabicyclo[2.2.2] octane, ou SR 140333, de formule : 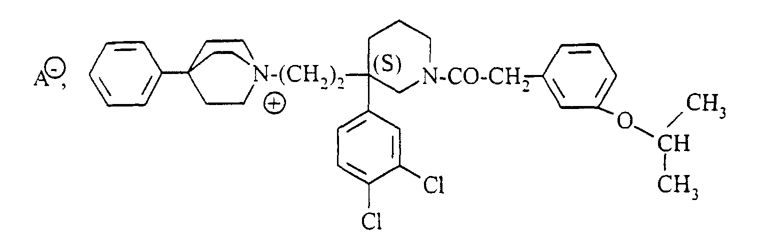 dans laquelle A^{⊖} est un anion pharmaceutiquement acceptable.

Le benzènesulfonate du SR 140333 ci-après dénommé composé A, est tout particulièrement préféré. La dénomination commune internationale de ce composé est bésilate de nolpitantium.

Les composés de formule (I) ont été décrits comme des antagonistes de la substance P, ligand naturel des récepteurs NK₁ et ont donc une affinité pour lesdits récepteurs. Pour administrer de tels composés par voie orale, il est nécessaire qu'ils présentent une bonne absorption, ce qui implique à la fois une bonne solubilité en milieu aqueux et une bonne capacité à traverser la membrane intestinale. (M. Rowland and T.N. Tozer in Clinical pharmacokinetics, concepts and applications, Lea and Fehiger ed., 1989, 2nd edition, p. 113-130).

La solubilité des composés de formule (I) a été étudiée dans différents milieux : leur solubilité dans l'eau est généralement inférieure à 5 mg/ml mais ils sont solubles dans les solvants hydrophiles, tels que les alcools ou les glycols.

Les composés de formule (I) étant des ammonium quaternaires, ils restent sous forme ionisée quel que soit le pH du milieu dans lequel ils se trouvent, notamment à pH neutre qui est le pH du milieu intestinal.

Il est connu que les composés ioniques, notamment les ammonium quaternaires, traversent difficilement les membranes épithéliales (J.P. Labaune dans Pharmacocinétique, Principes fondamentaux, Masson ed., 1988, 2nd edition, p. 7-33).

La lignée cellulaire Caco-2 a la particularité de se différencier *in vitro* pour former une monocouche épithéliale. Cette lignée est classiquement utilisée pour évaluer la perméabilité épithéliale des composés (Crit. Rev. Ther. Drug Carrier System, 1991, 8 (4), 105-330).

Sur ce modèle, la perméabilité du composé A mis en solution dans le diméthylsulfoxyde (DMSO) est de 3,4. 10⁻⁷ cm/s. Par ailleurs, des études réalisées, chez le rat, avec le composé A en solution aqueuse à 0,6 % dans la méthylcellulose, ont montré que son absorption estimée est inférieure à 1 %.

Des tensioactifs non ioniques et des agents améliorant l'absorption ont été testés pour connaitre leur effet sur la perméabilité membranaire d'un composé de formule (I). On a constaté que ces produits n'ont aucun effet positif.

De plus, les composés de formule (I) présentent d'autres propriétés physicochimiques qui rendent leur formulation difficile. Ainsi, la faible densité des composés de formule (I) est peu compatible avec leur formulation par simple mélange à sec. Par ailleurs, ces composés étant très électrostatiques, ils ont tendance à coller aux surfaces, ce qui est nuisible lors d'éventuels processus de compression.

En ce qui concerne le composé A, il est cristallisé sous forme d'aiguilles et son fort caractère électrostatique conduit à la formation d'aggrégats. Par ailleurs, la micronisation du produit serait potentiellement dangereuse à cause du risque d'explosion par accumulation de charges.

Ainsi pour mettre au point une formulation galénique pour les composés de formule (I) il faut à la fois remédier à leur très faible solubilité et à leur faible passage des membranes, et parvenir à une formulation compatible avec la préparation de formes sèches.

On a maintenant trouvé que l'on peut préparer une formulation pharmaceutique présentée sous forme sèche pour administration orale d'un composé de formule (I) en utilisant la granulation humide pour formuler le produit ; de telles formulations pharmaceutiques permettent à la fois d'éviter l'accumulation de charges électriques et d'améliorer la biodisponibilité du principe actif.

Par granulation humide on entend l'opération pharmaceutique qui permet, à l'aide d'un liquide de granulation, de densifier un mélange de poudres contenant le principe actif, ledit mélange constituant la phase interne de la formulation, la masse humide ainsi obtenue étant séchée puis calibrée avant l'addition des ingrédients constituant la phase externe de ladite formulation.

De façon surprenante, on a observé que l'addition d'un polysorbate particulier, le polysorbate 80, au composé A en solution dans le milieu de culture des cellules, améliore nettement le passage transépithélial sur le modèle Caco-2.

Les formulations pharmaceutiques selon l'invention contiennent de 10 mg à 100 mg de polysorbate 80 par unité de dosage. Plus particulièrement, les formulations pharmaceutiques selon l'invention contiennent de 15 mg à 60 mg de polysorbate 80 par unité de dosage.

Les formulations pharmaceutiques selon la présente invention peuvent se présenter sous forme de gélules, de comprimés, de sachets ou de poudres.

Pour les compositions pharmaceutiques sous forme de gélules ou de comprimés, on peut également préparer des formulations entériques.

De telles formulations sont utilisées pour protéger le principe actif de la forte acidité stomacale. De telles formulations sont préparées par enrobage de la gélule ou du comprimé par un film de polymère insoluble dans un environnement acide et soluble dans un environnement basique.

Le diluant utilisé dans la composition de la présente invention peut être un ou plusieurs composés qui sont capables de densifier le principe actif pour obtenir la masse désirée. Les diluants préférés sont les phosphates minéraux, tels que les phosphates de calcium ; les sucres, tels le lactose hydraté ou anhydre, le mannitol ; et la cellulose ou les dérivés cellulosiques comme par exemple la cellulose microcristalline, l'amidon, l'amidon de maïs ou l'amidon prégélatinisé. Tout particulièrement, on préfère le lactose monohydrate, le mannitol, la cellulose microcristalline et l'amidon de maïs utilisés seuls ou en mélange ; par exemple un mélange de lactose monohydrate et d'amidon de maïs ou un mélange de lactose monohydrate, d'amidon de maïs et de cellulose microcristalline.

Le liant employé dans la composition de la présente invention peut être un ou plusieurs composés qui sont capables de densifier un composé de formule (I) en le transformant en particules plus grosses et plus denses, ayant un meilleur écoulement. Les liants préférés sont l'acide alginique, l'alginate de sodium ; la cellulose et les dérivés cellulosiques, tels que la carboxyméthylcellulose de sodium, l'éthylcellulose, l'hydroxyéthylcellulose, l'hydroxypropylcellulose, l'hydroxypropylméthylcellulose ou la méthylcellulose ; la gélatine ; les polymères d'acide acrylique ; la povidone par exemple la povidone K-30 ; l'hydroxypropylméthylcellulose et la povidone K-30 étant des liants tout particulièrement préférés.

L'agent de désagrégation employé dans la composition de la présente invention peut être un ou plusieurs composés qui facilitent la désagrégation de la formulation préparée quand elle est placée dans un milieu aqueux. Les agents de désagrégation préférés sont la cellulose ou les dérivés cellulosiques, tels que carboxyméthylcellulose de sodium, carboxyméthylcellulose de sodium réticulée, cellulose microcristalline, cellulose en poudre, crospovidone ; l'amidon prégélatinisé, le glyconate d'amidon sodique, le carboxyméthylamidon de sodium ou l'amidon. La crospovidone, la carboxyméthylcellulose de sodium réticulée et le carboxyméthylamidon de sodium sont des agents de désagrégation préférés.

L'antiadhérent employé dans la composition de la présente invention peut être un ou plusieurs composés qui sont capables de réduire le caractère collant de la formulation, par exemple d'empêcher l'adhérence aux surfaces métalliques. Les antiadhérents préférés sont des composés qui contiennent du silicium, par exemple de la silice ou du talc.

L'agent d'écoulement employé dans la composition de la présente invention peut être un ou plusieurs composés qui sont capables de faciliter l'écoulement de la formulation préparée. Les agents d'écoulement préférés sont des composés qui contiennent du silicium, par exemple de la silice colloïdale anhydre ou de la silice précipitée.

Le lubrifiant employé dans la composition de la présente invention peut être un ou plusieurs composés qui sont capables d'empêcher les problèmes liés à la préparation de formes sèches, comme les problèmes de collage et/ou de grippage qui surviennent au niveau des machines lors de la compression ou du remplissage. Les lubrifiants préférés sont des acides gras ou des dérivés d'acides gras comme le stéarate de calcium, le monostéarate de glycéryle, le palmitostéarate de glycéryle, le stéarate de magnésium, le laurylsulfate de sodium, le stéarylfumarate de sodium, le stéarate de zinc, ou l'acide stéarique ; les huiles végétales hydrogénées, par exemple l'huile de ricin hydrogénée ; les polyalkylèneglycols, le polyéthylèneglycol ; le benzoate de sodium ou le talc. Selon la présente invention, le stéarate de magnésium ou le stéarylfumarate de sodium sont préférés.

Le colorant employé dans la formulation de la présente invention peut être un ou plusieurs composés qui sont capables de donner la couleur souhaitée à la formulation préparée. L'addition d'un colorant peut servir par exemple à distinguer des formulations ayant des dosages différents en principe actif. Les colorants préférés sont des oxydes de fer.

A titre d'exemple de film pour l'enrobage entérique on peut citer l'acétophtalate de cellulose, l'acétophtalate de polyvinyle, le phtalate d'hydroxypropylméthyl cellulose ou des copolymères d'acide méthacrylique.

A titre d'exemple de copolymère d'acide méthacrylique, on peut citer le copolymère d'acide méthacrylique de type C, commercialisé sous le nom de marque EUDRAGIT® L30 D-55 par ROHM ou le copolymère d'acrylate d'éthyle et d'acide méthacrylique commercialisé sous le nom de marque KOLLICOAT® MAE 30D par BASF.

Pour augmenter l'élasticité du film d'enrobage, on peut ajouter des plastifiants, tels que par exemple : un polyéthylèneglycol, le 1,2-propylèneglycol, le dibutyl phtalate ou un citrate.

Dans certains cas, en particulier lorsque l'on prépare des formulations entériques avec des gélules de gélatine, il peut être préférable de recouvrir la gélule par un pelliculage constitué d'un préenrobage avant l'enrobage entérique. Le préenrobage peut être fait par exemple avec de l'hydroxypropylcéllulose, de la povidone ou un copolymère d'acide méthacrylique associé à des excipients appropriés.

Selon la présente invention, les formulations pharmaceutiques sont préparées par un procédé de granulation humide caractérisé en ce que :
a) pour la phase interne, on mélange à température ambiante le principe actif, le diluant, éventuellement le liant, le polysorbate 80, et éventuellement le colorant ;
b) on mouille avec l'eau purifiée comme liquide de granulation ;
c) on sèche puis on calibre la masse humide ainsi obtenue ;
d) aux grains secs calibrés ainsi obtenus, on ajoute les ingrédients de phase externe à savoir le lubrifiant, l'agent de désagrégation, l'antiadhérent, l'agent d'écoulement et éventuellement le colorant et/ou l'aromatisant.

Selon la présente invention, un mode de réalisation particulier consiste à incorporer le polysorbate 80 dans l'eau purifiée pour procéder à la granulation humide.

Selon un autre mode de réalisation particulier, on incorpore le liant dans l'eau purifiée pour procéder à la granulation humide.

Lorsque l'on prépare des formulations entériques, le pelliculage d'une gélule ou d'un comprimé selon l'invention peut contenir un film d'enrobage ayant la composition suivante (en poids) :

| Enrobage : | |
|---|---|
| copolymère d'acide méthacrylique de type C | 54,8 % |
| glycérine | 3,3 % |
| solution de polysorbate 80 à 33 % | 0,7 % |
| eau | 41,2 % |

Pour le pelliculage de gélules entériques, il est préférable de procéder d'abord à un préenrobage avec un film ayant la composition suivante (en poids) :

| Préenrobage : | |
|---|---|
| copolymère d'acide méthacrylique de type C | 46,6 % |
| glycérine | 4,6 % |
| solution aqueuse de polysorbate 80 à 33 % | 4,6 % |
| eau | 44,2 % |

Plus particulièrement, la présente invention est relative à une formulation pharmaceutique pour administration orale d'un composé de formule (I) contenant en poids :

| | |
|---|---|
| principe actif | 0,5 à 20 % |
| liant | 2,5 à 6 % |
| agent de désagrégation | 0 à 5 % |
| antiadhérent | 0 à 3 % |
| lubrifiant | 0,5 à 3 % |
| agent d'écoulement | 0 à 5 % |
| polysorbate 80 | 4 à 20 % |
| colorant | 0 à 2 % |
| aromatisant | 0 à 2 % |
| diluant en quantité suffisante (Q.S.) pour 100 % | |

Selon un mode de réalisation particulier, la présente invention concerne une formulation pharmaceutique pour administration orale contenant en poids :

| | |
|---|---|
| composé A | 0,5 à 10 % |
| lactose monohydrate | 60 à 80 % |
| amidon de maïs | 15 à 25 % |
| povidone K-30 | 2 à 5 % |
| polysorbate 80 | 4 à 20 % |
| stéarate de magnésium | 1 % |

Les compositions pharmaceutiques selon l'invention contiennent de 10 à 100 mg et plus particulièrement de 15 mg à 60 mg de polysorbate 80 par unité de dosage. Ce composé est selon un mode de réalisation préféré, ajouté avec l'eau pour la granulation.

Ainsi la présente invention a tout spécialement pour objet la formulation pharmaceutique pour administration orale d'un composé A ayant la formulation suivante :

| dans la phase interne : | |
|---|---|
| composé A | 3,1 % |
| lactose monohydrate | 66,6 % |
| amidon de maïs | 20 % |
| povidone K-30 | 3 % |
| eau purifiée pour granulation humide Q.S. | |
| polysorbate 80 | 6,3 % |

| dans la phase externe : | |
|---|---|
| stéarate de magnésium | 1 %. |

Dans cette formulation le polysorbate 80 peut être incorporé à l'eau pour granulation.

Les caractéristiques et avantages des compositions ci-dessus apparaitront à la lumière de la description ci-après, à partir des compositions données à titre d'exemples.

### ESSAIS

### 1. Solubilité du composé A

La solubilité dans l'eau a été mesurée à saturation, après 24 heures, à température ambiante ; les mesures ont été réalisées par spectrométrie U.V. à λ = 275 nm après étalonnage dans une solution éthanolique.

| pH | 1 | 3 | 5 | 7 | 9 |
|---|---|---|---|---|---|
| Concentration en composé A mg/ml | 0,31 | 0,31 | 0,31 | 0,29 | 0,31 |

On voit bien que cette solubilité est faible et non dépendante du pH.

Par ailleurs, le composé A est soluble dans le diméthylsulfoxyde (DMSO) à 168 mg/ml ;

### 2. Evaluation du passage transépithélial intestinal du composé A.

Sur des filtres microporeux en polycarbonate recouverts de collagène, on ensemence des cellules Caco-2. La monocouche cellulaire formée sur le filtre permet alors de séparer un compartiment apical (mimant la lumière intestinale) d'un compartiment basal (mimant la circulation sanguine).

On place alors du côté apical la composition contenant le composé à étudier et on évalue le passage de ce composé, dispersé ou solubilisé dans le milieu de Hank, à travers cette barrière cellulaire, en mesurant sa cinétique d'apparition du côté basal. Ce milieu aqueux, de pH = 6,5, a la composition suivante : NaCl = 8,0 g/l ; KCl = 0,4 g/l ; CaCl₂ = 0,19 g/l ; MgCl₂ = 0,1 g/l ; MgSO₄ = 0,1 g/l ; Na₂HPO₄ = 0,09 g/l ; KH₂PO₄ = 0,06 g/l ; NaHCO₃ = 0,35 g/l ; glucose = 1 g/l ; rouge phénol = 0,01 g/l.

On a étudié le passage transépithélial de la formulation 1 suivante, réalisée par granulation humide selon l'invention :

| Formulation 1 | |
|---|---|
| composé A | 0,7 % |
| lactose monohydrate | 75,3 % |
| amidon de maïs | 20 % |
| povidone K-30 | 3 % |
| stéarate de magnésium | 1 % |

Sur le modèle Caco-2, la vitesse de passage du composé A dans la formulation 1 est multipliée par 3 par rapport à celle du composé A en suspension dans le milieu de culture des cellules. Ainsi, grâce à la formulation 1, la vitesse de passage du composé A est semblable à celle mesurée lorsque le composé A est en solution dans le DMSO. La formulation 1 permet donc de corriger totalement la caractéristique de faible solubilité du composé A.

A la formulation décrite ci-dessus, placée dans le milieu de Hank, on a ajouté différents agents tensioactifs non ioniques ou des agents réputés pour améliorer l'absorption tels qu'un acide gras en C₈-C₁₀ ou un de ses dérivés et on a mesuré la vitesse relative du passage transépithélial intestinal du composé A dans ces formulations.

| Formulation | Concentration de l'agent ajouté (mg/l) | Vitesse de transport relative |
|---|---|---|
| Formulation 1 | 0 | 1 ± 0,20 |
| 1 + polysorbate 60 | 100 | 0,51 ± 0,22 |
| | 200 | 0,62 ± 0,15 |
| 1 + polysorbate 80 | 100 | 2,77 ± 1,53 |
| | 200 | 4,39 ± 0,25 |
| 1 + Cremophor® RH 40 | 100 | 0,66 ± 0,40 |
| | 200 | 0,51 ± 0,18 |
| 1 + Synperonic® 44 | 100 | 0,92 ± 0,10 |
| | 200 | 1,48 ± 0,24 |
| 1 + Synperonic® 127 | 100 | 0,85 ± 0,06 |
| | 200 | 1,19 ± 0,17 |
| 1 + Span® 20 | 100 | 0,73 ± 0,48 |
| | 200 | 0,60 ± 0,28 |
| 1 + acide caprylique | 100 | 0,39 ± 0,03 |
| | 200 | 0,34 ± 0,01 |
| 1 + monocapryloyl glycérol | 100 | 0,27 ± 0,06 |
| | 200 | 0,56 ± 0,11 |

Dans les différentes formulations étudiées, le composé A étant dissous par les constituants de la formulation 1, c'est bien l'effet des différents agents sur l'absorption du composé A qui est mesuré.

Seul le polysorbate 80 (100 et 200 mg/l) ajouté à la formulation 1 provoque une augmentation notoire de la vitesse de transport du composé A, alors que les autres tensioactifs non ioniques ou les agents améliorant l'absorption, soit n'ont pas d'effet, soit diminuent la vitesse de transport.

Par ailleurs, on a vérifié que le polysorbate 80 ne provoque pas d'altération de la membrane épithéliale, et ce jusqu'à une concentration élevée (4800 mg/l).

Selon les données de la littérature, le volume intestinal est d'environ 250 ml (J.B. Dressmann et al., J. Pharm. Sci., 1985, 74, 588-589). Dans les tests effectués ci-dessus, la concentration de l'agent dans le milieu de Hank correspond donc à la quantité dudit agent qui serait présente par unité de dosage de la formulation. Ainsi les concentrations de polysorbate 80 de 100 mg/l et 200 mg/l correspondent respectivement à des quantités de 25 mg et 50 mg par unité de dosage.

### 3. Evaluation in vivo de l'absorption du composé A.

Chez le chien anesthésié, on induit une hypotension par des administrations successives de [Sar⁹,Met(O₂)¹¹] substance P, par voie i.v. à la dose de 5 ng/kg et on étudie l'effet inhibiteur de cette hypotension par administration du composé A dans différentes formulations.

Par voie intra-duodénale, l'administration du composé A en solution à la dose de 0,35 mg/kg permet de rétablir la pression artérielle alors que son administration en suspension à la dose de 0,35 mg/kg n'a aucun effet sur la pression artérielle.

Par "solution", on entend que le composé A est dans le sérum physiologique après dissolution par de l'éthanol ou du DMSO. Par "suspension", on entend que le composé A est dans une solution aqueuse contenant 6 % de méthylcellulose.

A la dose de 1 mg/kg, par voie orale (p.o.), le composé A permet de rétablir la pression artérielle s'il est administré en solution, alors qu'il n'a aucun effet sur la pression s'il est administré pur dans une gélule.

On a également administré le composé A pur en gélule à la dose 3 mg/kg p.o. ; et à la dose de 10 mg/kg p.o. L'effet sur la pression artérielle est faible à 3 mg/kg tandis qu'il est maximal à 10 mg/kg.

Enfin, on a administré au chien anesthésié le composé A formulé selon la formulation 1 de la présente invention, à la dose de 3 mg/kg et on a constaté que l'effet sur la pression artérielle est identique à celui que l'on observe lorsque le composé A est administré à 1 mg/kg p.o. en solution dans l'éthanol ou dans le DMSO.

Ces résultats montrent que l'effet inhibiteur du composé A vis-à-vis de l'hypotension induite par la [Sar⁹,Met(O₂)¹¹] substance P, se manifeste seulement lorsque le composé A est administré en solution.

On voit que la formulation 1 permet de diviser par 3 la dose active du composé A. Ceci montre l'intérêt d'une telle formulation pour solubiliser le composé A et pour améliorer son absorption intestinale.

Dans la présente description et dans les exemples ci-après les quantités des ingrédients sont exprimés en pourcentage en poids par rapport au poids total de la formulation pharmaceutique.

### EXEMPLE 1 : Gélules

| | | |
|---|---|---|
| composé A | 12,5 mg soit | 3,1 % |
| lactose monohydrate | 262,7 mg | 66,6 % |
| amidon de maïs | 79 mg | 20 % |
| povidone K-30 | 11,85 mg | 3 % |
| polysorbate 80 | 25 mg | 6,3 % |
| stéarate de magnésium | 3,95 mg | 1 % |
| pour une gélule de taille 0 terminée à | 395 mg | |

### EXEMPLE 2 : Gélules

| | | |
|---|---|---|
| composé A | 6,25 mg soit | 1,6 % |
| lactose monohydrate | Q.S. | 69,4 % |
| amidon de maïs | 79 mg | 20 % |
| polysorbate 80 | 20 mg | 5 % |
| povidone K-30 | 11,85 mg | 3 % |
| stéarate de magnésium | 3,95 mg | 1 % |
| pour une gélule de taille 0 terminée à | 395 mg | |

### EXEMPLE 3 : Gélules

| | | |
|---|---|---|
| composé A | 62,5 mg soit | 15,6 % |
| lactose monohydrate | Q.S. | 50,3 % |
| amidon de maïs | 70 mg | 17,6 % |
| polysorbate 80 | 50 mg | 12,5 % |
| povidone K-30 | 12 mg | 3 % |
| stéarate de magnésium | 4 mg | 1 % |
| pour une gélule de taille 0 terminée à | 400 mg | |

### EXEMPLE 4 : Gélules

| | | |
|---|---|---|
| composé A | 6,25 mg soit | 1,4 % |
| mannitol | Q.S. | 89,2 % |
| polysorbate 80 | 20 mg | 4,4 % |
| hydroxypropylméthylcellulose | 13,5 mg | 3 % |
| carboxyméthylcellulose de sodium réticulée | 4,5 mg | 1 % |
| stéarate de magnésium | 4,5 mg | 1 % |
| pour une gélule de taille 0 terminée à | 450 mg | |

### EXEMPLE 5 : Gélules

| | | |
|---|---|---|
| composé A | 62,5 mg soit | 13,8 % |
| mannitol | Q.S. | 68,1 % |
| polysorbate 80 | 50 mg | 11,1 % |
| hydroxypropylméthylcellulose | 22,5 mg | 5 % |
| carboxyméthylcellulose de sodium réticulée | 4,5 mg | 1 % |
| stéarate de magnésium | 4,5 mg | 1 % |
| pour une gélule de taille 0 terminée à | 450 mg | |

### EXEMPLE 6: Comprimés

| | | |
|---|---|---|
| composé A | 6,25 mg soit | 2,1 % |
| lactose monohydrate | Q.S. | 69,7 % |
| amidon de maïs | 50 mg | 16,6 % |
| povidone K-30 | 9 mg | 3 % |
| polysorbate 80 | 20 mg | 6,6 % |
| carboxyméthylamidon de sodium | 3 mg | 1 % |
| stéarate de magnésium | 3 mg | 1 % |
| pour un comprimé terminé à | 300 mg | |

### EXEMPLE 7 : Comprimés

| | | |
|---|---|---|
| composé A | 6,25 mg soit | 2,1 % |
| lactose monohydrate | Q.S. | 74,3 % |
| povidone K-30 | 9 mg | 3 % |
| polysorbate 80 | 50 mg | 16,6 % |
| carboxyméthylamidon de sodium | 3 mg | 1 % |
| silice colloïdale anhydre | 6 mg | 2 % |
| stéarate de magnésium | 3 mg | 1 % |
| pour un comeprimé terminé à | 300 mg | |

### EXEMPLE 8 : Comprimés

| | | |
|---|---|---|
| composé A | 62,5 mg soit | 12,5 % |
| lactose monohydrate | Q.S. | 52,5 % |
| amidon de maïs | 50 mg | 10 % |
| cellulose microcristalline | 50 mg | 10 % |
| hydroxypropylcellulose | 25 mg | 5 % |
| polysorbate 80 | 20 mg | 4 % |
| polyplasdone | 15 mg | 3 % |
| stéarylfumarate de sodium | 15 mg | 3 % |
| pour un comprimé terminé à | 500 mg | |

### EXEMPLE 9 : Comprimés

| | | |
|---|---|---|
| composé A | 62,5 mg soit | 12,5 % |
| lactose monohydrate | Q.S. | 73,9 % |
| polysorbate 80 | 50 mg | 10 % |
| carboxyméthylamidon sodique | 3 mg | 0,6 % |
| silice colloïdale anhydre | 10 mg | 2 % |
| stéarate de magnésium | 5 mg | 1 % |
| pour un comprimé terminé à | 500 mg | |

### EXEMPLE 10 : Sachets

| | | |
|---|---|---|
| composé A | 100 mg soit | 10% |
| lactose monohydrate | 610 mg | 61 % |
| amidon de maïs | 200 mg | 20 % |
| polysorbate 80 | 50 mg | 5 % |
| carboxyméthylcellulose de sodium réticulée | 30 mg | 3 % |
| stéarate de magnésium | 10 mg | 1 % |
| pour un sachet rempli à | 1000 mg | |

### EXEMPLE 11 : Gélules entériques

On prépare une gélule selon l'exemple 1 et on applique un pelliculage en 2 couches : l'une de préenrobage et l'autre d'enrobage.

| Préenrobage : | |
|---|---|
| Eudragit® L30 D-55 | 46,6 % |
| glycérine | 4,6 % |
| solution aqueuse de polysorbate 80 à 33 % | 4,6 % |
| eau | 44,2 % |

| Enrobage : | |
|---|---|
| Eudragit® L30 D-55 | 54,8 % |
| glycérine | 3,3 % |
| solution aqueuse de polysorbate 80 à 33 % | 0,7 % |
| eau | 41,2 % |

## Revendications

1. Formulation pharmaceutique présentée sous forme sèche pour l'administration orale d'un principe actif de formule: 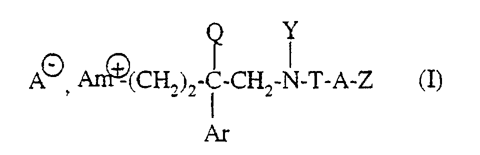 dans laquelle :
- A^{⊖} est un anion pharmaceutiquement acceptable ;
- Am^{⊕} représente :
i - soit un groupe Am₁^{⊕} de formule : 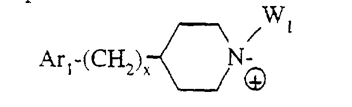 dans laquelle :
- Ar₁ représente un phényle non substitué ou substitué une ou plusieurs fois par un substituant choisi parmi un atome d'halogène, un hydroxy, un (C₁-C₄)alcoxy, un (C₁-C₄)alkyle ou un trifluorométhyle, lesdits substituants étant identiques ou différents ;
- x est zéro ou un ;
- W1 représente un (C₁-C₆)alkyle ou un groupe benzyle ; le substituant W₁ étant soit en position axiale soit en position équatoriale ;
ii - soit un groupe Am₂^{⊕} de formule : 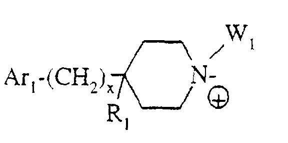 dans laquelle :
- Ar₁, x et W₁ sont tels que définis ci-dessus ;
- R₁ représente un hydroxy ; un (C₁-C₄)alcoxy ; un formyloxy ; un (C₁-C₃)alkylcarbonyloxy ; un carboxy ; un (C₁-C₄)alcoxycarbonyle; un cyano ; un (C₁-C₃)alkylcarbonylamino ; un mercapto ; un (C₁-C₄)alkylthio ;
iii - soit un groupe Am₃^{⊕} de formule : 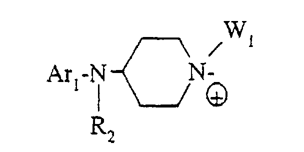 dans laquelle :
- Ar₁ et W₁ sont tels que définis ci-dessus ;
- R₂ représente l'hydrogène ; un (C₁-C₃)alkyle ; un (C₁-C₃)alkylcarbonyle ;
iv - soit un groupe Am₄^{⊕} de formule : 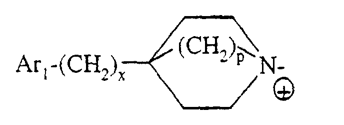 dans laquelle :
- Ar₁ et x sont tels que définis ci-dessus ;
- p est un ou deux ;
v - soit un groupe Am₅^{⊕} de formule: 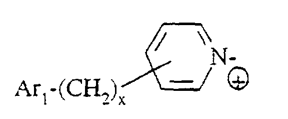 dans laquelle :
- Ar₁ et x sont tels que définis ci-dessus ;
- Ar représente un phényle non substitué ou substitué une ou deux fois par un substituant choisi parmi : un atome d'halogène, un (C₁-C₃)alcoxy, un (C₁-C₃)alkyle, un trifluorométhyle, lesdits substituants étant identiques ou différents ; un naphtyle ; un indolyle ;
- Q et Y représentent l'un des groupes de valeurs suivantes :
a) Q₁ et Y₁ ;
b) Q₂ et Y₂ lorsque Am^{⊕} représente un groupe Am₁^{⊕}, Am₂^{⊕}, Am₄^{⊕} ou Am₅^{⊕} ;
c) Q₃ et Y₃ lorsque Am^{⊕} représente un groupe Am₁^{⊕}, Am₂^{⊕} ou un groupe Am₄^{⊕} dans lequel Ar₁ représente un phényle et p est deux ;
d) Q₄ et Y₄ lorsque Am^{⊕} représente un groupe Am₁^{⊕}, Am₃^{⊕}, Am₄^{⊕} ou Am₅^{⊕} ;
- Q₁ représente l'hydrogène ;
- Y₁ représente l'hydrogène ; un (C₁-C₄)alkyle ; un ω-(C₁-C₄)alcoxy-(C₂-C₄)alkylène ; un ω-(C₁-C₄)alkylcarbonyloxy-(C₂-C₄)alkylène ; un ω-benzoyloxy-(C₂-C₄)alkylène; un ω-hydroxy-(C₂-C₄)alkylène ; un ω-(C₁-C₄)alkylthio-(C₂-C₄)alkylène ; un ω-(C₁-C₄)alkylcarbonyl-(C₂-C₄)alkylène ; un ω-carboxy-(C₂-C₄)alkylène ; un ω-(C₁-C₄)alcoxycarbonyl-(C₂-C₄)alkylène; un ω-benzyloxy-(C₂-C₄)alkylène ; un ω-formyloxy-(C₂-C₄)alkylène ; un ω-R₃NHCOO-(C₂-C₄)alkylène ; un ω-R₄R₅NCO-(C₂-C₄)alkylène ; un ω-R₆CONR₇-(C₂-C₄)alkylène ; un ω-R₈OCONR₇-(C₂-C₄)alkylène ; un ω-R₄R₅NCONR₇-(C₂-C₄)alkylène; un ω-R₉SO₂NR₇-(C₂-C₄)alkylène ; un ω-cyano-(C₁-C₃)alkylène;
- Q₂ et Y₂, ensemble, constituent un groupe éthylène, triméthylène ou tétraméthylène ;
- Q₃ et Y₃, ensemble, constituent un groupe : 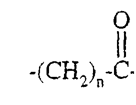 dans lequel n est un, deux ou trois ;
- Q₄ et Y₄, ensemble, constituent un radical choisi parmi :
A₁) -O-CH₂-
A₂) -O-CO-
A₃) -CH₂-O-CO-
A₄) -O-CH₂-CO-
A₅) -O-CH₂-CH₂-
A₆) -N(R₁₀)-CO-
A₇) -N(R₁₀)-CO-CO-
A₈) -N(R₁₀)-CH₂-CH₂-
- T représente - soit un groupe -CO- lorsque Q et Y représentent Q₁ et Y₁, Q₂ et Y₂ ou Q₄ et Y₄ lorsqu'ils constituent, ensemble, un radical A₁), A₅) ou A₈) ;
- soit un groupe -CH₂- lorsque Q et Y représentent Q₃ et Y₃ ou Q₄ et Y₄ lorsqu'ils constituent, ensemble, un radical A₂), A₃), A₄), A₆) ou A₇) ;
- A représente soit une liaison directe ou un groupe méthylène lorsque T est -CO-, soit une liaison directe lorsque T est -CH₂- ;
- Z représente :
- un phényle non substitué ou substitué une ou plusieurs fois par un substituant choisi parmi un atome d'halogène ; un trifluorométhyle ; un cyano ; un hydroxy ; un nitro ; un amino non substitué ou substitué une ou plusieurs fois par un (C₁-C₄)alkyle ; un benzylamino ; un carboxy ; un (C₁-C₁₀)alkyle ; un (C₃-C₇)cycloalkyle non substitué ou substitué une ou plusieurs fois par un méthyle ; un (C₁-C₁₀)alcoxy ; un (C₃-C₇)cycloalkyloxy non substitué ou substitué une ou plusieurs fois par un méthyle ; un mercapto ; un (C₁-C₁₀)alkylthio ; un (C₁-C₆)alkylcarbonyloxy; un (C₁-C₆)alkylcarbonylamino; un benzoylamino ; un (C₁-C₄)alcoxycarbonyle ; un (C₃-C₇)cycloalkylcarbonyle ; un carbamoyle non substitué ou substitué une ou deux fois par un (C₁-C₄)alkyle ; un uréido non substitué ou substitué une ou deux fois en position 3 par un (C₁-C₄)alkyle ou un (C₃-C₇)cycloalkyle ; un (pyrrolidin-1-yl)carbonylamino, lesdits substituants étant identiques ou différents ;
- un naphtyle non substitué ou substitué une ou plusieurs fois par un halogène, un trifluorométhyle, un (C₁-C₄)alkyle, un hydroxy, un (C₁-C₄)alcoxy ;
- un pyridyle ; un thiényle ; un indolyle ; un quinolyle ; un benzothiényle ; un imidazolyle ;
- R₃ représente un (C₁-C₇)alkyle ou un phényle ;
- R₄ et R₅ représentent chacun indépendamment un hydrogène ou un (C₁-C₇)alkyle ; R₅ peut de plus représenter un (C₃-C₇)cycloalkyle, un (C₃-C₇)cycloalkylméthyle, un phényle ou un benzyle ; ou bien R₄ et R₅ ensemble avec l'atome d'azote auquel ils sont liés constituent un hétérocycle choisi parmi l'azétidine, la pyrrolidine, la pipéridine, la morpholine, la thiomorpholine, la perhydroazépine et la pipérazine non substituée ou substituée en position 4 par un (C₁-C₄)alkyle ;
- R₆ représente un hydrogène, un (C₁-C₇)alkyle, un vinyle, un phényle, un benzyle, un pyridyle ou un (C₃-C₇)cycloalkyle non substitué ou substitué par un ou plusieurs méthyles ;
- R₇ représente un hydrogène ou un (C₁-C₇)alkyle ;
- R₈ représente un (C₁-C₇)alkyle ou un phényle ;
- R₉ représente un (C₁-C₇)alkyle ; un amino non substitué ou substitué par un ou deux (C₁-C₇)alkyles ; un phényle non substitué ou substitué une ou plusieurs fois par un substituant choisi parmi un atome d'halogène, un (C₁-C₇)alkyle, un trifluorométhyle, un hydroxy, un (C₁-C₇)alcoxy, un carboxy, un (C₁-C₇)alcoxycarbonyle, un (C₁-C₇)alkylcarbonyloxy, un cyano, un nitro, un amino non substitué ou substitué par un ou deux (C₁-C₇)alkyles, lesdits substituants étant identiques ou différents ;
- R₁₀ représente l'hydrogène ou un (C₁-C₄)alkyle ;
ou d'un de ses sels éventuels avec des acides minéraux ou organiques et d'un de leurs solvates éventuels,
formulée par granulation humide, contenant en pourcentage du poids total de la formulation :
- principe actif 0,5 à 50 %
- liant 1 à 10 %
- agent de désagrégation 0 à 10 %
- antiadhérant 0 à 5 %
- lubrifiant 0,2 à 5 %
- agent d'écoulement 0 à 15 %
- polysorbate 80 4 à 20 %
- colorant 0 à 2 %
- aromatisant 0 à 2 %
- diluant en quantité suffisante (Q.S.) pour 100 %, et **caractérisée en ce qu'**elle contient de 10 mg à 100 mg de polysorbate 80 par unité de dosage.

2. Formulation pharmaceutique selon la revendication 1 **caractérisée en ce que** le principe actif est un composé de formule : 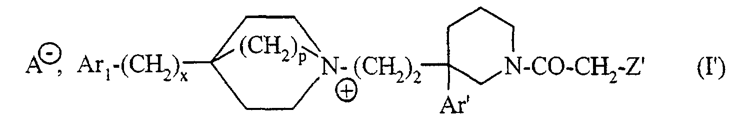 dans laquelle :
- Ar₁, x et p sont tels que définis pour un composé de formule (I) dans la revendication 1 ;
- Ar' représente un 3,4-dichlorophényle ou un 3,4-difluorophényle ;
- Z' représente un phényle substitué en position 3 par un halogène ou un (C₁-C₁₀)alcoxy ;
- A^{⊖} représente un anion pharmaceutiquement acceptable.

3. Formulation pharmaceutique selon l'une quelconque des revendications 1 ou 2, **caractérisée en ce que** le principe actif est le (S)-1-{2-[3-(3,4-dichlorophényl)-1-(3-isopropoxyphénylacétyl)pipéridin-3-yl]éthyl }-4-phényl-1-azoniabicyclo[2.2.2] octane, de formule : 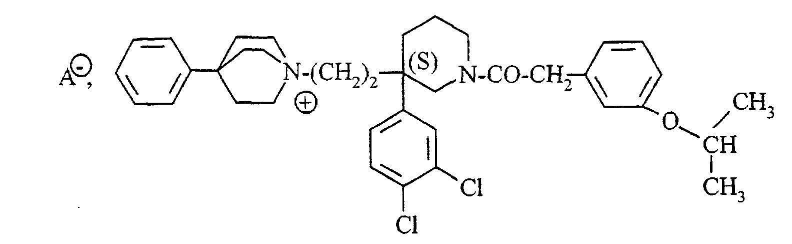 dans laquelle A^{⊖} est un anion pharmaceutiquement acceptable.

4. Formulation pharmaceutique selon l'une quelconque des revendications 1 à 3, **caractérisée en ce que** le principe actif est le bésilate de nolpitantium.

5. Formulation pharmaceutique selon l'une quelconque des revendications 1 à 4, **caractérisée en ce qu'**elle contient de 15 mg à 60 mg de polysorbate 80 par unité de dosage.

6. Formulation pharmaceutique selon l'une quelconque des revendications 1 à 5, **caractérisée en ce qu'**elle se présente sous forme de gélules, de comprimés, de sachets ou de poudres.

7. Formulation pharmaceutique selon l'une quelconque des revendications 1 à 6, **caractérisée en ce que** le diluant est un composé ou un mélange de composés choisi parmi les phosphates de calcium, le lactose hydraté ou anhydre, le mannitol, la cellulose microcristalline, l'amidon, l'amidon de maïs ou l'amidon prégélatinisé.

8. Formulation pharmaceutique selon la revendication 7, **caractérisée en ce que** le diluant est un composé ou un mélange de composés choisi parmi le lactose monohydrate, le mannitol, la cellulose microcristalline et l'amidon de maïs.

9. Formulation pharmaceutique selon l'une des revendications 7 ou 8, **caractérisée en ce que** le diluant est un mélange de lactose monohydrate et d'amidon de maïs ou un mélange de lactose monohydrate, d'amidon de maïs et de cellulose microcristalline.

10. Formulation pharmaceutique selon l'une quelconque des revendications 1 à 9, **caractérisée en ce que** le liant est un composé ou un mélange de composés choisi parmi l'acide alginique, l'alginate de sodium ; la cellulose, la carboxyméthylcellulose de sodium, l'éthylcellulose, l'hydroxyéthylcellulose, l'hydroxypropylcellulose, l'hydroxypropylméthylcellulose ou la méthylcellulose ; la gélatine ; les polymères d'acide acrylique ; la povidone K-30.

11. Formulation pharmaceutique selon la revendication 10, **caractérisée en ce que** le liant est choisi parmi l'hydroxypropylméthylcellulose et la povidone K-30.

12. Formulation pharmaceutique selon l'une quelconque des revendications 1 à 11. **caractérisée en ce que** l'agent de désagrégation est un composé ou un mélange de composés choisi parmi la cellulose, la carboxyméthylcellusose de sodium, la carboxyméthylcellulose de sodium réticulée, la cellulose microcristalline, la cellulose en poudre, la crospovidone ; l'amidon prégélatinisé, le glyconate d'amidon sodique, le carboxyméthylamidon de sodium et l'amidon.

13. Formulation pharmaceutique selon la revendication 12, **caractérisée en ce que** l'agent de désagrégation est choisi parmi la crospovidone, la carboxyméthylcellulose de sodium réticulée et le carboxyméthylamidon de sodium.

14. Formulation pharmaceutique selon l'une quelconque des revendications 1 à 13, **caractérisée en ce que** l'antiadhérent est choisi parmi la silice et le talc.

15. Formulation pharmaceutique selon l'une quelconque des revendications 1 à 14, **caractérisée en ce que** l'agent d'écoulement est choisi parmi la silice colloïdale anhydre et la silice précipitée.

16. Formulation pharmaceutique selon l'une quelconque des revendications 1 à 15, **caractérisée en ce que** le lubrifiant est un composé ou un mélange de composés choisis parmi le stéarate de calcium, le monostéarate de glycéryle, le palmitostéarate de glycéryle, le stéarate de magnésium, le laurylsulfate de sodium, le stéarylfumarate de sodium, le stéarate de zinc, ou l'acide stéarique ; l'huile de ricin hydrogénée, les polyalkylèneglycols, le polyéthylèneglycol, le benzoate de sodium et le talc.

17. Formulation pharmaceutique selon la revendication 16, **caractérisée en ce que** le lubrifiant est choisi parmi le stéarate de magnésium et le stéarylfumarate de sodium.

18. Formulation pharmaceutique selon l'une quelconque des revendications 1 à 17, contenant :
| | |
|---|---|
| principe actif | 0,5 à 20 % |
| liant | 2,5 à 6 % |
| agent de désagrégation | 0 à 5 % |
| antiadhérent | 0 à 3 % |
| lubrifiant | 0,5 à 3 % |
| agent d'écoulement | 0 à 5 % |
| polysorbate 80 | 4 à 20 % |
| colorant | 0 à 2 % |
| aromatisant | 0 à 2 % |
| diluant en quantité suffisante (Q.S.) pour 100 % | |

19. Formulation pharmaceutique selon l'une quelconque des revendications 1 à 18, contenant :
| | |
|---|---|
| bésilate de nolpitantium | 0,5 à 10 % |
| lactose monohydrate | 60 à 80 % |
| amidon de maïs | 15 à 25 % |
| povidone K-30 | 2 à 5 % |
| polysorbate 80 | 4 à 20 % |
| stéarate de magnésium | 1 % |

20. Formulation pharmaceutique selon la revendication 19 contenant :
| dans la phase interne : | |
|---|---|
| bésilate de nolpitantium | 3,1 % |
| lactose monohydrate | 66,6 % |
| amidon de maïs | 20 % |
| povidone K-30 | 3 % |
| eau purifiée pour granulation humide Q.S. | |
| polysorbate 80 | 6,3 % |
| dans la phase externe : | |
|---|---|
| stéarate de magnésium | 1 %. |

21. Formulation pharmaceutique selon l'une quelconque des revendications 1 à 20 pour la préparation de gélules entériques.

22. Formulation pharmaceutique selon l'une quelconque des revendications 1 à 20 pour la préparation de comprimés entériques.

23. Procédé pour la préparation de formulations pharmaceutiques selon l'une quelconque des revendications 1 à 22, **caractérisée en ce que** :
a) pour la phase interne on mélange à température ambiante le principe actif, le diluant, éventuellement le liant ;
b) on mouille à l'eau purifiée ;
c) on sèche et on calibre la masse humide ainsi obtenue ;
d) aux grains secs calibrés ainsi obtenus on ajoute les ingrédients de phase externe à savoir le lubrifiant, l'agent de désagrégation, l'antiadhérent, l'agent d'écoulement, et **en ce que** l'on incorpore en outre du polysorbate 80 soit à l'étape a) soit à l'étape b).

24. Procédé selon la revendication 23, **caractérisé en ce que** l'on incorpore en outre un colorant à l'étape a) ou à l'étape d).

25. Procédé selon l'une des revendications 23 ou 24 **caractérisé en ce que** l'on incorpore en outre un aromatisant à l'étape d).

26. Procédé selon l'une quelconque des revendications 23 à 25, **caractérisée en ce que** à l'étape b) on incorpore le polysorbate 80 dans l'eau purifiée.

27. Procédé selon l'une quelconque des revendications 23 à 26, **caractérisée en ce que** à l'étape b) on incorpore le liant dans l'eau purifiée au lieu de l'incorporer à l'étape a).

28. Procédé selon l'une quelconque des revendications 23 à 27 pour la préparation de formulations entériques.

## Patentansprüche

1. Pharmazeutische Formulierung in trockener Form zur oralen Verabreichung eines Wirkstoffs der Formel 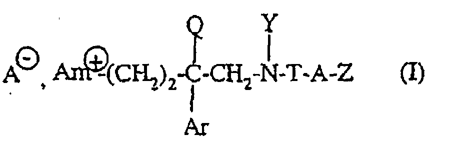 worin:
- A⁻ ein pharmazeutisch annehmbares Anion ist;
- Am⁺ folgendes darstellt:
i - entweder eine Gruppe Am₁⁺ der Formel: 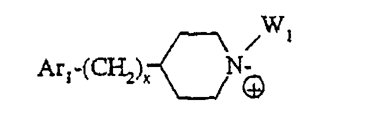 worin:
- Ar₁ ein gegebenenfalls ein- oder mehrmals durch einen Substituenten, ausgewählt aus einem Halogenatom, Hydroxy, (C₁-C₄)-Alkoxy, (C₁-C₄)-Alkyl oder Trifluormethyl, substituiertes Phenyl darstellt, wobei die Substituenten gleich oder verschieden sind;
- x null oder eins ist;
- W₁ ein (C₁-C₆)-Alkyl oder eine Benzylgrupe darstellt, wobei der Substituent W₁ entweder in der axialen oder in der äquatorialen Position steht;
ii - oder eine Gruppe Am₂⁺ der Formel: 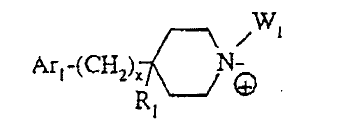 worin:
- Ar₁, x und W₁ wie oben definiert sind;
- R₁ Hydroxy; (C₁-C₄)-Alkoxy; Formyloxy; (C₁-C₃)-Alkylcarbonyloxy; Carboxy; (C₁-C₄)-Alkoxycarbonyl; Cyano; (C₁-C₃)-Alkylcarbonylamino; Mercapto; (C₁-C₄)-Alkylthio darstellt;
iii - oder eine Gruppe Am₃⁺ der Formel: 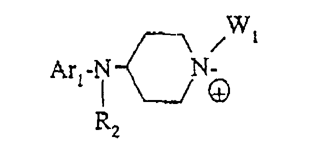 worin:
- Ar₁, x und W₁ wie oben definiert sind;
- R₂ Wasserstoff; (C₁-C₃)-Alkyl oder (C₁-C₃)-Alkylcarbonyl darstellt;
iv - oder eine Gruppe Am₄⁺ der Formel: 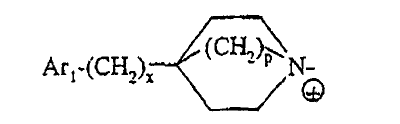 worin:
- Ar₁ und x wie oben definiert sind;
- p eins oder zwei ist;
v - oder eine Gruppe Am₅⁺ der Formel: 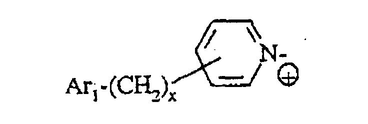 worin:
- Ar₁ und x wie oben definiert sind;
- Ar ein nicht oder ein- oder zweimal durch einen Substituenten ausgewählt aus einem Halogenatom; (C₁-C₃)-Alkoxy; (C₁-C₃)-Alkyl oder Trifluormethyl, substituiertes Phenyl darstellt, wobei die Substituenten gleich oder verschieden sind; Naphthyl oder Indolyl;
- Q und Y eine der Gruppen mit folgenden Werten darstellen:
a) Q₁ und Y₁,
b) Q₂ und Y₂, wenn Am⁺ eine Gruppe Am₁⁺, Am₂⁺, Am₄⁺ oder Am₅⁺ darstellt;
c) Q₃ und Y₃, wenn Am⁺ eine Gruppe Am₁⁺, Am₂⁺ oder eine Gruppe Am₄⁺ darstellt, in der Ar₁ Phenyl darstellt und p zwei ist;
d) Q₄ und Y₄, wenn Am⁺ eine Gruppe Am₁⁺, Am₃⁺, Am₄⁺ oder Am₅⁺ darstellt;
- Q₁ Wasserstoff darstellt;
- Y₁ Wasserstoff; (C₁-C₄)-Alkyl; ω-(C₁-C₄)-Alkoxy-(C₂-C₄)-Alkylen; ω-(C₁-C₄)-Alkyl-carbonyloxy-(C₂-C₄)-Alkylen; ω-Benzoyloxy-(C₂-C₄)-alkylen; ω-(C₁-C₄)-Hydroxy-(C₂-C₄)-alkylen; ω-(C₁-C₄)-Alkylthio-(C₂-C₄)-alkylen; ω-(C₁-C₄)-Alkylcarbonyl-(C₂-C₄)-Alkylen; ω-Carboxy-(C₂-C₄)-alkylen; ω-(C₁-C₄)-Alkoxycarbonyl-(C₂-C₄)-alkylen; ω-Benzyloxy-(C₂-C₄)-alkylen; ω-Formyloxy-(C₂-C₄)-alkylen; ω-R₃NHCOO-(C₂-C₄)-alkylen; ω-R₄R₅NCO-(C₂-C₄)-alkylen; ω-R₆CONR₇-(C₂-C₄)-alkylen; ω-R₈OCONR₇-(C₂-C₄)-alkylen; ω-R₄R₅CONR₇-(C₂-C₄)- alkylen; ω-R₉SO₂NR₇-(C₂-C₄)-alkylen; ω-Cyano-(C₁-C₃)-alkylen darstellt;
- Q₂ und Y₂ zusammen eine Ethylen-, Trimethylen- oder Tetramethylengruppe bilden;
- Q₃ und Y₃ zusammen eine Gruppe: 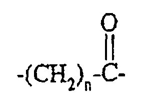 bilden, worin n eins; zwei oder drei ist;
- Q₄ und Y₄ zusammen einen Rest bilden, ausgewählt aus:
A₁) -O-CH₂-
A₂) -O-CO-
A₃) -CH₂-O-CO-
A₄) -O-CH₂-CO-
A₅) -O-CH₂-CH₂-
A₆) -N(R₁₀)-CO-
A₇) -N(R₁₀)-CO-CO-
A₈) -N(R₁₀)-CH₂-CH₂-
- T - entweder eine Gruppe -CO- darstellt, wenn Q und Y für Q₁ und Y₁, Q₂ und Y₂ oder Q₄ und Y₄ stehen, wenn sie zusammen einen Rest A₁), A₅) oder A₈) bilden;
- oder eine Gruppe -CH₂- darstellt, wenn Q und Y für Q₃ und Y₃ oder Q₄ und Y₄ stehen, wenn sie zusammen einen Rest A₂), A₃), A₄), A₆) oder A₇) bilden;
- A entweder eine direkte Bindung oder eine Methylengruppe darstellt, wenn T für -CO- steht, oder eine direkte Bindung, wenn T für -CH₂- steht;
- Z folgendes darstellt:
- ein Phenyl, das gegebenenfalls ein- oder mehrmals durch einen Substituenten substituiert ist, ausgewählt aus einem Halogenatom; Trifluormethyl; Cyano; Hydroxy; Nitro; einem gegebenenfalls ein- oder mehrmals durch (C₁-C₄)-Alkyl substituierten Amino; Benzylamino; Carboxy; (C₁-C₁₀)-Alkyl; einem gegebenenfalls ein- oder mehrmals durch ein Methyl substituierten (C₃-C₇)-Cycloalkyl; (C₁-C₁₀)-Alkoxy; einem gegebenenfalls ein- oder mehrmals durch ein Methyl substituierten (C₃-C₇)-Cycloalkyloxy; Mercapto; (C₁-C₁₀)-Alkylthio; (C₁-C₆)-Alkylcarbonyloxy; (C₁-C₆)-Alkylcarbonylamino; Benzoylamino; (C₁-C₄)-Alkoxycarbonyl; (C₃-C₇)-Cycloalkylcarbonyl; einem gegebenenfalls ein- oder zweimal durch (C₁-C₄)-Alkyl substituierten Carbamoyl; einem gegebenenfalls ein- oder zweimal in Position 3 durch (C₁-C₄)-Alkyl oder (C₃-C₇)-Cycloalkyl substituierten Ureido, (Pyrrolidin-1-yl)-carbonylamino, wobei die Substituenten gleich oder verschieden sind;
- ein gegebenenfalls ein- oder mehrmals durch ein Halogen, Trifluormethyl, (C₁-C₄)-Alkyl, Hydroxy oder (C₁-C₄)-Alkoxy substituiertes Naphthyl;
- Pyridyl; Thienyl; Indolyl; Chinolyl; Benzothienyl oder Imidazolyl;
- R₃ (C₁-C₇)-Alkyl oder Phenyl darstellt;
- R₄ und R₅ jeweils unabhängig Wasserstoff oder (C₁-C₇)-Alkyl darstellen; R₅ weiters (C₃-C₇)-Cycloalkyl, (C₃-C₇)-Cycloalkylmethyl, Phenyl oder Benzyl darstellen kann; oder aber es bilden R₄ und R₅ zusammen mit dem Stickstoffatom, an das sie gebunden sind, einen Heterocyclus ausgewählt aus Azetidin, Pyrrolidin, Piperidin, Morpholin, Thiomorpholin, Perhydroazepin und Piperazin, gegebenenfalls in Position 4 durch (C₁-C₄)-Alkyl substituiert, bilden;
- R₆ Wasserstoff, (C₁-C₇)-Alkyl, Vinyl, Phenyl, Benzyl, Pyridyl oder (C₃-C₇)-Cycloalkyl, gegebenenfalls durch ein oder mehrere Methyle substituiert, darstellt;
- R₇ Wasserstoff oder (C₁-C₇)-Alkyl darstellt;
- R₈ (C₁-C₇)-Alkyl oder Phenyl darstellt;
- R₉ (C₁-C₇)-Alkyl; ein gegebenenfalls durch ein oder zwei (C₁-C₇)-Alkyle substituiertes Amino; ein gegebenenfalls ein- oder mehrmals durch einen Substituenten, ausgewählt aus einem Halogenatom, (C₁-C₇)-Alkyl, Trifluormethyl, Hydroxy, (C₁-C₇)-Alkoxy, Carboxy, (C₁-C₇)-Alkoxycarbonyl, (C₁-C₇)-Alkylcarbonyloxy, Cyano, Nitro, einem gegebenenfalls durch ein oder zwei (C₁-C₇)-Alkyle substituierten Amino, substituiertes Phenyl darstellt, wobei die Substituenten gleich oder verschieden sind;
- R₁₀ Wasserstoff oder (C₁-C₄)-Alkyl darstellt;
oder eines seiner möglichen Salze mit Mineral- oder organischen Säuren und eines der möglichen Solvate derselben,
formuliert durch Feuchtgranulation, enthaltend in Prozent des Gesamtgewichts der Formulierung:
- Wirkstoff 0,5 bis 50 %
- Bindemittel 1 bis 10 %
- Zerfallsförderndes Mittel 0 bis 10 %
- Antihaftmittel 0 bis 5 %
- Gleitmittel 0,2 bis 5 %
- Fließmittel 0 bis 15 %
- Polysorbat 80 4 bis 20 %
- Farbstoff 0 bis 2 %
- Aromastoff 0 bis 2 %
- Verdünnungsmittel in ausreichender Menge (q.s.) auf 100 %
und **dadurch gekennzeichnet, dass** sie 10 mg bis 100 mg Polysorbat 80 pro Dosiseinheit enthält.

2. Pharmazeutische Formulierung nach Anspruch 1, **dadurch gekennzeichnet, dass** der Wirkstoff eine Verbindung der Formel: 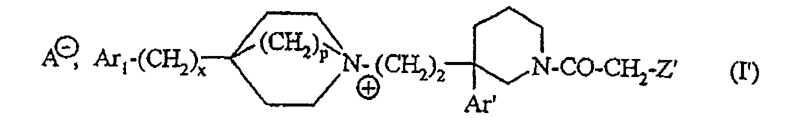 ist, worin:
- Ar₁, x und p wie für eine Verbindung der Formel (I) im Anspruch 1 definiert sind;
- Ar' 3,4-Dichlorphenyl oder 3,4-Difluorphenyl darstellt;
- Z' ein in Position 3 durch ein Halogen oder (C₁-C₁₀)-Alkoxy substituiertes Phenyl darstellt;
- A⁻ ein pharmazeutisch annehmbares Anion darstellt;

3. Pharmazeutische Formulierung nach einem der Ansprüche 1 oder 2, **dadurch gekennzeichnet, dass** der Wirkstoff (S)-1-{2-[3-(3,4-Dichlorphenyl)-1-(3-isopropoxyphenylacetyl)piperidin-3-yl]ethyl}-4-phenyl-1-azoniabicyclo[2.2.2]octan der Formel: 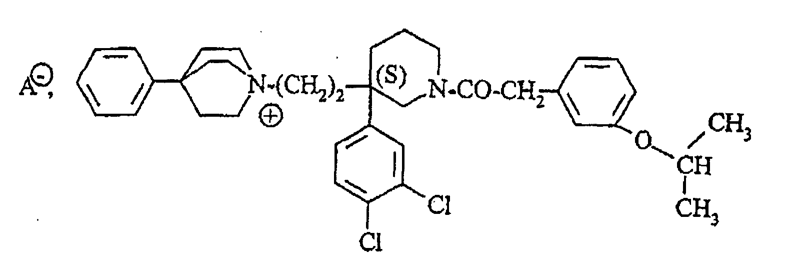 ist, worin A⁻ ein pharmazeutisch annehmbares Anion ist.

4. Pharmazeutische Formulierung nach einem der Ansprüche 1 bis 3, **dadurch gekennzeichnet, dass** der Wirkstoff Nolpitantiumbesilat ist.

5. Pharmazeutische Formulierung nach einem der Ansprüche 1 bis 4, **dadurch gekennzeichnet, dass** sie 15 mg bis 60 mg Polysorbat 80 pro Dosiseinheit enthält.

6. Pharmazeutische Formulierung nach einem der Ansprüche 1 bis 5, **dadurch gekennzeichnet, dass** sie in Form von Kapseln, Tabletten, Säckchen oder Pulvern vorliegt.

7. Pharmazeutische Formulierung nach einem der Ansprüche 1 bis 6, **dadurch gekennzeichnet, dass** das Verdünnungsmittel eine Verbindung oder eine Mischung von Verbindungen, ausgewählt aus Calciumphosphaten, wasserhaltiger oder wasserfreier Lactose, Mannit, mikrokristalliner Cellulose, Stärke, Maisstärke oder vorgelatinierter Stärke, ist.

8. Pharmazeutische Formulierung nach Anspruch 7, **dadurch gekennzeichnet, dass** das Verdünnungsmittel eine Verbindung oder eine Mischung von Verbindungen, ausgewählt aus Lactosemonohydrat, Mannit, mikrokristalliner Cellulose und Maisstärke, ist.

9. Pharmazeutische Formulierung nach Anspruch 7 oder 8, **dadurch gekennzeichnet, dass** das Verdünnungsmittel eine Mischung von Lactosemonohydrat und Maisstärke oder eine Mischung von Lactosemonohydrat, Maisstärke und mikrokristalliner Cellulose ist.

10. Pharmazeutische Formulierung nach einem der Ansprüche 1 bis 9, **dadurch gekennzeichnet, dass** das Bindemittel eine Verbindung oder eine Mischung von Verbindungen, ausgewählt aus Algininsäure, Natriumalginat; Cellulose, Natriumcarboxymethylcellulose, Ethylcellulose, Hydroxyethylcellulose, Hydroxypropylcellulose, Hydroxypropylmethylcellulose oder Methylcellulose; Gelatine; Acrylsäurepolymeren oder Povidon K-30, ist.

11. Pharmazeutische Formulierung nach Anspruch 10, **dadurch gekennzeichnet, dass** das Bindemittel ausgewählt aus Hydroxypropylmethylcellulose und Povidon K-30 ist.

12. Pharmazeutische Formulierung nach einem der Ansprüche 1 bis 11, **dadurch gekennzeichnet, dass** das zerfallsfördernde Mittel eine Verbindung oder eine Mischung von Verbindungen, ausgewählt aus Cellulose, Natriumcarboxymethylcellulose, vernetzter Natriumcarboxymethylcellulose, mikrokristalliner Cellulose, Cellulose in Pulverform, Crospovidon; vorgelatinierter Stärke, Natriumstärkeglyconat, Natriumcarboxymethylstärke und Stärke, ist.

13. Pharmazeutische Formulierung nach Anspruch 12, **dadurch gekennzeichnet, dass** das zerfallsfördernde Mittel ausgewählt aus Crospovidon, vernetzter Natriumcarboxymethylcellulose und Natriumcarboxymethylstärke ist.

14. Pharmazeutische Formulierung nach einem der Ansprüche 1 bis 13, **dadurch gekennzeichnet, dass** das Antihaftmittel ausgewählt aus Kieselsäure und Talk ist.

15. Pharmazeutische Formulierung nach einem der Ansprüche 1 bis 14, **dadurch gekennzeichnet, dass** das Fließmittel ausgewählt aus kolloidaler wasserfreier Kieselsäure und Kieselgallerte ist.

16. Pharmazeutische Formulierung nach einem der Ansprüche 1 bis 15, **dadurch gekennzeichnet, dass** das Gleitmittel eine Verbindung oder eine Mischung von Verbindungen, ausgewählt aus Calciumstearat, Glycerylmonostearat, Glycerylpalmitostearat, Magnesiumstearat, Natriumlaurylsulfat, Natriumstearylfumarat, Zinkstearat oder Stearinsäure; hydriertem Ricinusöl, Polyalkylenglycolen, Polyethylenglycol, Natriumbenzoat und Talk, ist.

17. Pharmazeutische Formulierung nach Anspruch 16, **dadurch gekennzeichnet, dass** das Gleitmittel ausgewählt aus Magnesiumstearat und Natriumstearylfumarat ist.

18. Pharmazeutische Formulierung nach einem der Ansprüche 1 bis 17, enthaltend:
| | |
|---|---|
| Wirkstoff | 0,5 bis 20 % |
| Bindemittel | 2,5 bis 6 % |
| Zerfallsförderndes Mittel | 0 bis 5 % |
| Antihaftmittel | 0 bis 3 % |
| Gleitmittel | 0,5 bis 3 % |
| Fließmittel | 0 bis 5 % |
| Polysorbat 80 | 4 bis 20 % |
| Farbstoff | 0 bis 2 % |
| Aromastoff | 0 bis 2 % |
| Verdünnungsmittel in ausreichender Menge (q.s.) auf 100 %. | |

19. Pharmazeutische Formulierung nach einem der Ansprüche 1 bis 18, enthaltend:
| | |
|---|---|
| Nolpitantiumbesilat | 0,5 bis 10 % |
| Lactosemonohydrat | 60 bis 80 % |
| Maisstärke | 15 bis 25 % |
| Povidon K-30 | 2 bis 5 % |
| Polysorbat 80 | 4 bis 20 % |
| Magnesiumstearat | 1 % |

20. Pharmazeutische Formulierung nach Anspruch 19, enthaltend
| in der inneren Phase: | |
|---|---|
| Nolpitantiumbesilat | 3,1 % |
| Lactosemonohydrat | 66,6 % |
| Maisstärke | 20 % |
| Povidon K-30 | 3 % |
| Gereinigtes Wasser für Feuchtgranulation q.s. | |
| Polysorbat 80 | 6,3 % |
| in der äußeren Phase: | |
|---|---|
| Magnesiumstearat | 1 %. |

21. Pharmazeutische Formulierung nach einem der Ansprüche 1 bis 20 zur Herstellung von im Darm löslichen Kapseln.

22. Pharmazeutische Formulierung nach einem der Ansprüche 1 bis 20 zur Herstellung von im Darm löslichen Tabletten.

23. Verfahren zur Herstellung von pharmazeutischen Formulierungen nach einem der Ansprüche 1 bis 22, **dadurch gekennzeichnet, dass**:
a) für die innere Phase der Wirkstoff, das Verdünnungsmittel und gegebenenfalls das Bindemittel bei Umgebungstemperatur gemischt werden;
b) mit gereinigtem Wasser angefeuchtet wird;
c) die so erhaltene Masse getrocknet und kalibriert wird;
d) die so erhaltenen trockenen Körnchen mit den Ingredienzien der äußeren Phase, nämlich dem Gleitmittel, dem zerfallsfördernden Mittel, dem Antihaftmittel und dem Fließmittel versetzt werden,
und dass weiters Polysorbat 80 entweder in Schritt a) oder in Schritt b) eingearbeitet wird.

24. Verfahren nach Anspruch 23, **dadurch gekennzeichnet, dass** weiters ein Farbstoff in Schritt a) oder in Schritt d) eingearbeitet wird.

25. Verfahren nach einem der Ansprüche 23 oder 24, **dadurch gekennzeichnet, dass** weiters ein Aromastoff in Schritt d) eingearbeitet wird.

26. Verfahren nach einem der Ansprüche 23 bis 25, **dadurch gekennzeichnet, dass** in Schritt b) Polysorbat 80 in gereinigtem Wasser eingearbeitet wird.

27. Verfahren nach einem der Ansprüche 23 bis 26, **dadurch gekennzeichnet, dass** das Bindemittel in gereinigtem Wasser in Schritt b) und nicht in Schritt a) eingearbeitet wird.

28. Verfahren nach einem der Ansprüche 23 bis 27 zur Herstellung von im Darm löslichen Formulierungen.

## Claims

1. A pharmaceutical formulation, presented in dry form, for the oral administration of an active principle of the formula: 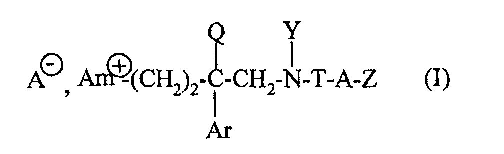 in which:
- A^{⊖} is a pharmaceutically acceptable anion;
- Am^{⊕} is:
i - either a group Am₁^{⊕} of the formula: 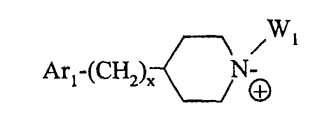 in which:
- Ar₁ is a phenyl which is unsubstituted or monosubstituted or polysubstituted by a substituent selected from a halogen atom, a hydroxyl, a (C₁-C₄)alkoxy, a (C₁-C₄)alkyl or a trifluoromethyl, said substituents being identical or different;
- x is zero or one;
- W₁ is a (C₁-C₆)alkyl or a benzyl group; the substituent W₁ being either in the axial position or in the equatorial position;
ii - or a group Am₂^{⊕} of the formula: 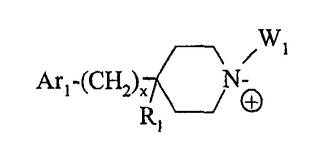 in which:
- Ar₁, x and W₁ are as defined above; and
- R₁ is a hydroxyl; a (C₁-C₄)alkoxy; a formyloxy; a (C₁-C₃)alkylcarbonyloxy; a carboxyl; a (C₁-C₄)alkoxycarbonyl; a cyano; a (C₁-C₃)alkylcarbonylamino; a mercapto; a (C₁-C₄)alkylthio;
iii - or a group Am₃^{⊕} of the formula: 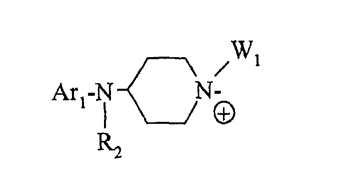 in which:
- Ar₁ and W₁ are as defined above;
- R₂ is hydrogen; a (C₁-C₃)alkyl; a (C₁-C₃)alkylcarbonyl;
iv - or a group Am₄^{⊕} of the formula: 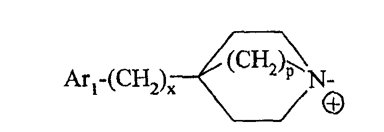 in which:
- Ar₁ and x are as defined above; and
- p is one or two;
v - or a group Am₅^{⊕} of the formula: 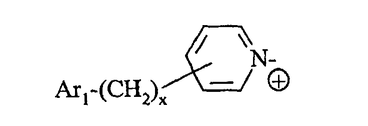 in which:
- Ar₁ and x are as defined above;
- Ar is a phenyl which is unsubstituted or monosubstituted or disubstituted by a substituent selected from a halogen atom, a (C₁-C₃)alkoxy, a (C₁-C₃)alkyl and a trifluoromethyl, said substituents being identical or different; a naphthyl; an indolyl;
- Q and Y have one of the following groups of meanings:
a) Q₁ and Y₁;
b) Q₂ and Y₂ when Am^{⊕} is a group Am₁^{⊕}, Am₂^{⊕}, Am₄^{⊕} or Am₅^{⊕};
c) Q₃ and Y₃ when Am^{⊕} is a group Am₁^{⊕} or Am₂^{⊕} or a group Am₄^{⊕} in which Ar₁ is a phenyl and p is two;
d) Q₄ and Y₄ when Am^{⊕} is a group Am₁^{⊕}, Am₃^{⊕}, Am₄^{⊕} or Am₅^{⊕};
- Q₁ is hydrogen;
- Y₁ is hydrogen; a (C₁-C₄)alkyl; an ω-(C₁-C₄)alkoxy-(C₂-C₄)alkylene; an ω-(C₁-C₄)alkylcarbonyloxy-(C₂-C₄)alkylene; an ω-benzoyloxy-(C₂-C₄)alkylene; an ω-hydroxy-(C₂-C₄)alkylene; an ω-(C₁-C₄)alkylthio-(C₂-C₄)alkylene; an ω-(C₁-C₄)alkylcarbonyl-(C₂-C₄)alkylene; an ω-carboxy-(C₂-C₄)alkylene; an ω-(C₁-C₄)alkoxycarbonyl-(C₂-C₄)alkylene; an ω-benzyloxy-(C₂-C₄)alkylene; an ω-formyloxy-(C₂-C₄)alkylene; an ω-R₃NHCOO-(C₂-C₄)alkylene; an ω-R₄R₅NCO-(C₂-C₄)alkylene; an ω-R₆CONR₇-(C₂-C₄)alkylene; an ω-R₈OCONR₇-(C₂-C₄)alkylene; an ω-R₄R₅NCONR₇-(C₂-C₄)alkylene; an ω-R₉SO₂NR₇-(C₂-C₄)alkylene; an ω-cyano(C₁-C₃)alkylene;
- Q₂ and Y₂ together form an ethylene, trimethylene or tetramethylene group;
- Q₃ and Y₃ together form a group 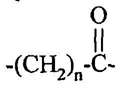 in which n is one, two or three;
- Q₄ and Y₄ together form a radical selected from:
A₁) -O-CH₂-
A₂) -O-CO-
A₃) -CH₂-O-CO-
A₄) -O-CH₂-CO-
A₅) -O-CH₂-CH₂-
A₆) -N(R₁₀)-CO-
A₇) -N(R₁₀)-CO-CO-
A₈) -N(R₁₀)-CH₂-CH₂-
- T is - either a group -CO- when Q and Y are Q₁ and Y₁, Q₂ and Y₂ or Q₄ and Y₄ when they together form a radical A₁), A₅) or A₈);
- or a group -CH₂- when Q and Y are Q₃ and Y₃ or Q₄ and Y₄ when they together form a radical A₂), A₃), A₄), A₆) or A₇);
- A is either a direct bond or a methylene group when T is -CO-, or a direct bond when T is -CH₂-;
- Z is:
- a phenyl which is unsubstituted or monosubstituted or polysubstituted by a substituent selected from a halogen atom; a trifluoromethyl; a cyano; a hydroxyl; a nitro; an amino which is unsubstituted or monosubstituted or polysubstituted by a (C₁-C₄)alkyl; a benzylamino; a carboxyl; a (C₁-C₁₀)alkyl; a (C₃-C₇)cycloalkyl which is unsubstituted or monosubstituted or polysubstituted by a methyl; a (C₁-C₁₀)alkoxy; a (C₃-C₇)cycloalkoxy which is unsubstituted or monosubstituted or polysubstituted by a methyl; a mercapto; a (C₁-C₁₀)alkylthio; a (C₁-C₆)alkylcarbonyloxy; a (C₁-C₆)alkylcarbonylamino; a benzoylamino; a (C₁-C₄)alkoxycarbonyl; a (C₃-C₇)cycloalkylcarbonyl; a carbamoyl which is unsubstituted or monosubstituted or disubstituted by a (C₁-C₄)alkyl; a ureido which is unsubstituted or monosubstituted or disubstituted in the 3-position by a (C₁-C₄)alkyl or a (C₃-C₇)cycloalkyl; and a (pyrrolidin-1-yl)carbonylamino, said substituents being identical or different;
- a naphthyl which is unsubstituted or monosubstituted or polysubstituted by a halogen, a trifluoromethyl, a (C₁-C₄)alkyl, a hydroxyl or a (C₁-C₄)alkoxy;
- a pyridyl; a thienyl; an indolyl; a quinolyl; a benzothienyl; an imidazolyl;
- R₃ is a (C₁-C₇)alkyl or a phenyl;
- R₄ and R₅ are each independently a hydrogen or a (C₁-C₇)alkyl; R₅ can also be a (C₃-C₇)cycloalkyl, a (C₃-C₇)cycloalkylmethyl, a phenyl or a benzyl; or R₄ and R₅, together with the nitrogen atom to which they are bonded, form a heterocycle selected from azetidine, pyrrolidine, piperidine, morpholine, thiomorpholine, perhydroazepine and piperazine which is unsubstituted or substituted in the 4-position by a (C₁-C₄)alkyl;
- R₆ is a hydrogen, a (C₁-C₇)alkyl, a vinyl, a phenyl, a benzyl, a pyridyl or a (C₃-C₇)cycloalkyl which is unsubstituted or substituted by one or more methyls;
- R₇ is a hydrogen or a (C₁-C₇)alkyl;
- R₈ is a (C₁-C₇)alkyl or a phenyl;
- R₉ is a (C₁-C₇)alkyl; an amino which is unsubstituted or substituted by one or two (C₁-C₇)alkyls; a phenyl which is unsubstituted or monosubstituted or polysubstituted by a substituent selected from a halogen atom, a (C₁-C₇)alkyl, a trifluoromethyl, a hydroxyl, a (C₁-C₇)alkoxy, a carboxyl, a (C₁-C₇)alkoxycarbonyl, a (C₁-C₇)alkylcarbonyloxy, a cyano, a nitro and an amino which is unsubstituted or substituted by one or two (C₁-C₇)alkyls, said substituents being identical or different;
- R₁₀ is hydrogen or a (C₁-C₄)alkyl,
or one of its salts with mineral or organic acids, if appropriate, and one of their solvates, if appropriate,
formulated by wet granulation, containing the following as percentages of the total weight of the formulation:
| | |
|---|---|
| active principle | 0.5 to 50% |
| binder | 1 to 10% |
| disintegrating agent | 0 to 10% |
| antiadhesive | 0 to 5% |
| lubricant | 0.2 to 5% |
| flow promoter | 0 to 15% |
| polysorbate 80 | 4 to 20% |
| color | 0 to 2% |
| flavoring | 0 to 2% |
diluent in sufficient amount (QS) for 100% and **characterized in that** it contains from 10 mg to 100 mg of polysorbate 80 per dosage unit.

2. A pharmaceutical formulation according to claim 1, **characterized in that** the active principle is a compound of the formula: 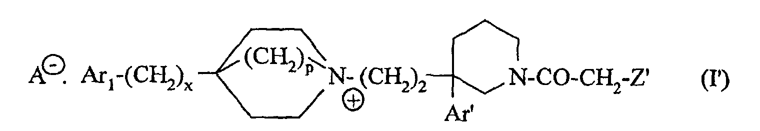 in which:
- Ar₁, x and p are as defined for a compound of formula (I) in claim 1;
- Ar' is a 3,4-dichlorophenyl or a 3,4-difluorophenyl;
- Z' is a phenyl substituted in the 3-position by a halogen or a (C₁-C₁₀)alkoxy; and
- A^{⊖} is a pharmaceutically acceptable anion.

3. A pharmaceutical formulation according to either one of claims 1 or 2, **characterized in that** the active principle is (S)-1-{2-[3-(3,4-dichlorophenyl)-1-(3-isopropoxyphenylacetyl)piperidin-3-yl]ethyl}-4-phenyl-1-azoniabicyclo[2.2.2]octane of the formula: 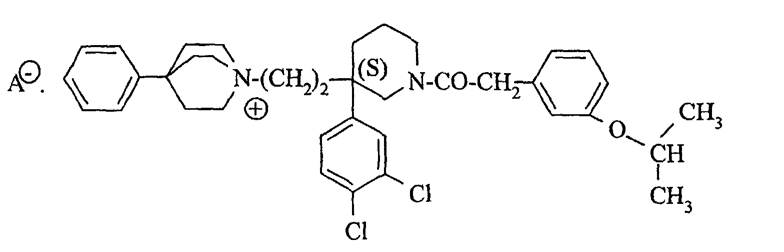 in which A^{⊖} is a pharmaceutically acceptable anion.

4. A pharmaceutical formulation according to any one of claims 1 to 3, **characterized in that** the active principle is nolpitantium besilate.

5. A pharmaceutical formulation according to any one of claims 1 to 4, **characterized in that** it contains from 15 mg to 60 mg of polysorbate 80 per dosage unit.

6. A pharmaceutical formulation according to any one of claims 1 to 5, **characterized in that** it is presented in the form of gelatin capsules, tablets, sachets or powders.

7. A pharmaceutical formulation according to any one of claims 1 to 6, **characterized in that** the diluent is a compound or a mixture of compounds selected from calcium phosphates, hydrated or anhydrous lactose, mannitol, microcrystalline cellulose, starch, corn starch or pregelatinized starch.

8. A pharmaceutical formulation according to claim 7, **characterized in that** the diluent is a compound or a mixture of compounds selected from lactose monohydrate, mannitol, microcrystalline cellulose and corn starch.

9. A pharmaceutical formulation according to one of claims 7 or 8, **characterized in that** the diluent is a mixture of lactose monohydrate and corn starch or a mixture of lactose monohydrate, corn starch and microcrystalline cellulose.

10. A pharmaceutical formulation according to any one of claims 1 to 9, **characterized in that** the binder is a compound or a mixture of compounds selected from alginic acid, sodium alginate; cellulose, sodium carboxymethyl cellulose, ethyl cellulose, hydroxyethyl cellulose, hydroxypropyl cellulose, hydroxypropyl methyl cellulose or methyl cellulose; gelatin; acrylic acid polymers; povidone K-30.

11. A pharmaceutical formulation according to claim 10, **characterized in that** the binder is selected from hydroxypropyl methyl cellulose and povidone K-30.

12. A pharmaceutical formulation according to any one of claims 1 to 11, **characterized in that** the disintegrating agent is a compound or a mixture of compounds selected from cellulose, sodium carboxymethyl cellulose, crosslinked sodium carboxymethyl cellulose, microcrystalline cellulose, cellulose powder or crospovidone; pregelatinized starch, sodium starch glyconate, sodium carboxymethyl starch and starch.

13. A pharmaceutical formulation according to claim 12, **characterized in that** the disintegrating agent is selected from crospovidone, crosslinked sodium carboxymethyl cellulose and sodium carboxymethyl starch.

14. A pharmaceutical formulation according to any one of claims 1 to 13, **characterized in that** the antiadhesive is selected from silica and talcum.

15. A pharmaceutical formulation according to any one of claims 1 to 14, **characterized in that** the flow promoter is selected from anhydrous colloidal silica and precipitated silica.

16. A pharmaceutical formulation according to any one of claims 1 to 15, **characterized in that** the lubricant is a compound or a mixture of compounds selected from calcium stearate, glyceryl monostearate, glyceryl palmitostearate, magnesium stearate, sodium laurylsulfate, sodium stearylfumarate, zinc stearate or stearic acid; hydrogenated castor oil, polyalkylene glycols, or polyethylene glycol, sodium benzoate and talcum.

17. A pharmaceutical formulation according to claim 16, **characterized in that** the lubricant is selected from magnesium stearate and sodium stearylfumarate.

18. A pharmaceutical formulation according to any one of claims 1 to 17 which contains:
| | |
|---|---|
| active principle | 0.5 to 20% |
| binder | 2.5 to 6% |
| disintegrating agent | 0 to 5% |
| antiadhesive | 0 to 3% |
| lubricant | 0.5 to 3% |
| flow promoter | 0 to 5% |
| polysorbate 80 | 4 to 20% |
| color | 0 to 2% |
| flavoring | 0 to 2% |
| diluent in sufficient amount (QS) for 100%. | |

19. A pharmaceutical formulation according to any one of claims 1 to 18 which contains:
| | |
|---|---|
| nolpitantium besilate | 0.5 to 10% |
| lactose monohydrate | 60 to 80% |
| corn starch | 15 to 25% |
| povidone K-30 | 2 to 5% |
| polysorbate 80 | 4 to 20% |
| magnesium stearate | 1%. |

20. A pharmaceutical formulation according to claim 19 which contains:
| in the internal phase: | |
|---|---|
| nolpitantium besilate | 3.1% |
| lactose monohydrate | 66.6% |
| corn starch | 20% |
| povidone K-30 | 3% |
| purified water for wet granulation QS | |
| polysorbate 80 | 6.3% |
| in the external phase: | |
|---|---|
| magnesium stearate | 1%. |

21. A pharmaceutical formulation according to any one of claims 1 to 20 for the preparation of enteric gelatin capsules.

22. A pharmaceutical formulation according to any one of claims 1 to 20 for the preparation of enteric tablets.

23. A process for the preparation of pharmaceutical formulations according to any one of claims 1 to 22, **characterized in that**:
a) for the internal phase, the active principle, the diluent and the optional binder are mixed at room temperature;
b) the mixture is wetted with purified water;
c) the resulting wet mass is dried and graded;
d) the ingredients of the external phase, namely the lubricant, the disintegrating agent, the antiadhesive and the flow promoter, are added to the graded dry grains obtained,
and **in that** polysorbate 80 is also incorporated, either in step a) or in step b).

24. A process according to claim 23, **characterized in that** a color is also incorporated in step a) or in step d).

25. A process according to one of claims 23 or 24, **characterized in that** a flavoring is also incorporated in step d).

26. A process according to any one of claims 23 to 25, **characterized in that** the polysorbate 80 is incorporated into the purified water in step b).

27. A process according to any one of claims 23 to 26, **characterized in that** the binder is incorporated into the purified water in step b) instead of being incorporated in step a).

28. A process according to any one of claims 23 to 27 for the preparation of enteric formulations.
